# EUROPÄISCHE PATENTANMELDUNG

(11) **EP 0 542 140 A2**
(43) Veröffentlichungstag der Anmeldung: **19.05.1993**
(21) Anmeldenummer: 92119002.1
(22) Anmeldetag: 06.11.1992
(51) Int. Cl.: A61M 1/14, G01N 27/07

(54) **Schlauchanordnung zur Verwendung in einem Blutkreislauf**

(30) Priorität: 15.11.1991 DE 4137628
(71) Anmelder: Fresenius AG, D-61350 Bad Homburg (DE)
(72) Erfinder: Polaschegg, Hans-Dietrich, Dr., W-6370 Oberursel 4 (DE)
(74) Vertreter: Fuchs, Luderschmidt & Partner Patentanwälte

(57) **Zusammenfassung**

Die Erfindung betrifft eine Schlauchanordnung zum Einsatz für Blutreinigungsverfahren, enthaltend Schlauchstücke mit wenigstens einer Elektrode zur Bestimmung von Parametern der den Schlauch durchströmenden Flüssigkeiten, die aus elektrisch leitendem Kunststoff besteht. Die Elektrode kann als vorgeformter Körper in eine Ausnehmung des Schlauchstücks eingefügt oder in situ in der Ausnehmung ausgehärtet werden. Alternativ können ringförmige Schlauchstücke abwechselnd aus leitfähigem und isolierendem Kunststoff gefertigt und miteinander verklebt werden. Die erfindungsgemäße Vorrichtung weist damit eine vollständig glatte Innenwand auf.

## Beschreibung

Die Erfindung betrifft eine Schlauchanordnung zum Einsatz für Blutreinigungsverfahren enthaltend Schlauchstücke mit wenigstens einer Elektrode zur Bestimmung von Parametern der den Schlauch durchströmenden Flüssigkeit.

Unter einer Schlauchanordnung zum Einsatz für Blutreinigungsverfahren werden aneinandergefügte Schläuche verstanden, die einerseits für den Blutkreislauf und andererseits für den Dialysierflüssigkeitskreislauf verwendet werden.

Im Verlauf einer Dialysebehandlung wird zum einen überschüssige Flüssigkeit aus dem Blutkreislauf des Patienten entfernt und zum anderen werden nicht abgebaute Stoffwechselprodukte ausgefiltert. Insbesondere bei der Entfernung überschüssiger Flüssigkeitsmengen durch die Ultrafiltration kann es beim Patienten zu Kopfschmerzen, Schwächegefühlen, Muskelkrämpfen und Erbrechen kommen (sog. Disäquilibriumssyndrom). Das Auftreten dieser Symptome wird u.a. darauf zurückgeführt, daß dem Blut zu schnell zu große Flüssigkeits- und damit Salz- bzw. Mineralstoffmengen, insbesondere Natrium, entzogen werden.

Zur verbesserten Kontrolle des Flüssigkeits- und Ionenentzugs aus dem Blut des Patienten wird daher nicht nur eine permanente Flüssigkeitsbilanzierung durchgeführt, sondern es wird das intravasale Blutvolumen kontinuierlich erfaßt.

Diese Erfassung der intravasalen Blutvolumens erfolgt durch die Einschaltung von Leitfähigkeits-Meßvorrichtungen, üblicherweise Elektroden, in den extrakorporalen Dialysierkreislauf. Die Elektroden und die Gehäuse, in die die Elektroden eingebaut sind und die vom Blut bzw. von der Dialysierflüssigkeit durchströmt werden, werden bisher aus Edelstahl gefertigt und in die Schlauchanordnungen des extrakorporalen Dialysekreislaufs eingefügt (Stiller und Mann, Biomedizinische Technik, 25, 1980, 286-289, Ergänzungsband).

Diese Materialzusammenstellung weist jedoch mehrere Nachteile auf. Zunächst wird kein biokompatibles Material für die Meßvorrichtung verwandt, zudem läßt sich das Leitfähigkeits-Meßelement mit dem zugehörigen Gehäuse nicht ohne - wenn auch geringfügige - Querschnittsänderungen in das Schlauchsystem und damit in die Flüssigkeitskreisläufe einfügen. Dies führt zu Strömungsverwirbelungen, die, beispielsweise im Blutkreislauf, die Gerinnungsneigung des Blutes deutlich heraufsetzen und damit die Belastung des Patienten durch die Dialyse erhöhen.

Neben diesen für den Dialyseablauf selbst bedeutsamen Nachteilen sind auch technische und wirtschaftliche Nachteile der bekannten Edelstahl-Vorrichtungen hinzunehmen. So ist zum einen die Herstellung einer solchen Vorrichtung aufwendig und durch die geforderten, präzise einzuhaltenden Toleranzvorgaben kostenintensiv, zum anderen wird für die Dialyse aus hygienischen Gründen die Verwendung von Einmal-Artikeln angestrebt. Die Edelstahlvorrichtungen sind als Einmal-Artikel jedoch außerordentlich teuer.

Aufgabe der vorliegenden Erfindung ist es daher, eine Leitfähigkeits-Meßvorrichtung bereitzustellen, die aus biokompatiblem Material preiswert herstellbar ist.

Die vorstehende Aufgabe wird gelöst, indem bei Blutreinigungsverfahren eine Schlauchanordnung zum Einsatz kommt, die Schlauchstücke mit wenigstens einer Elektrode zur Bestimmung von Parametern der den Schlauch durchströmenden Flüssigkeiten enthält, wobei die Elektrode aus einem elektrisch leitenden Kunststoff besteht.

Kunststoffe im Sinne des kennzeichnenden Merkmals des Anspruchs 1 sind natürliche oder synthetische ausgehärtete Polymere, beispielsweise Siliconkautschuk oder PVC. Sie werden durch Zusatz definierter Mengen leitfähigen Materials zu elektrisch leitenden Kunststoffen. Menge und Art des Zusatzes können gezielt gesteuert werden, so daß ein definierter Leitfähigkeits-Meßbereich den jeweiligen Anforderungen entsprechend eingestellt werden kann.

Die Eigenschaften elektrisch leitender Polymere, insbesondere elektrisch leitenden Silikonkautschuks, machen diese Materialien besonders geeignet für medizinische bzw. biomedizinische Verwendungszwecke. So läßt sich beispielsweise ein leitfähiges Elastomer mit einem vorbestimmten, genau eingestellten Durchgangswiderstand zur Messung im gewünschten Leitfähigkeitsbereich herstellen. In einer bevorzugten Ausführungsform der Erfindung wird für die Herstellung des Leitfähigkeitsmeßelements als Kunststoff Silikonkautschuk verwendet, der durch Zusatz von partikelförmigen Silber oder Kohlenstoff zu einem elektrisch leitfähigen Werkstoff wird.

Eine Elektrode aus solchem Material verhält sich biokompatibel und ist chemisch inert. Sie ist in der Herstellung zudem preiswerter als bekannte Vorrichtungen. Zudem läßt sich das leitfähige Polymer leichter verarbeiten als ein Metallkörper.

Die erfindungsgemäße Vorrichtung besteht weiter aus einem Schlauchstück aus isolierendem Kunststoff, das vorteilhafterweise als rohrförmiges Spritzgußteil, beispielsweise aus PVC oder Polycarbonat, hergestellt sein kann. In das Schlauchstück werden mindestens eine, üblicherweise jedoch zwei bis vier Öffnungen für Elektroden eingearbeitet, die die Wandung des Schlauchstücks durchbrechen. Es erleichtert die Handhabung beim Einfügen der Elektroden vorteilhaft, wenn das Schlauchstück als starrer, rohrförmiger Schlauchkörper ausgebildet ist.

In die Öffnungen in der Wandung des Schlauchstücks werden entweder vorgefertigte Elektrodenformkörper, die eine an die Wandung des Schlauchstückes angepaßte Meßfläche aufweisen, eingefügt, und mit geeigneten Mitteln (Klebe- bzw. Dichtmittel) flüssigkeitsdicht und paßgenau eingefügt. Die Elektrodenformkörper können über einen Anschluß mit einer Auswerteinheit zur Erfassung der gemessenen Werte und Umsetzung dieser Werte im Rahmen der Kontrolle des Dialysiervorgangs verbunden werden.

In einer weiteren Ausführungsform der Erfindung wird der Innenraum des Schlauchstücks mit einem Blindkörper mindestens im Bereich der Öffnung in der Wandung des Schlauchstücks vollständig ausgefüllt, wobei der Blindkörper vorteilhafterweise einen kreisförmigen Querschnitt aufweist, so daß sich die einzupassende Elektrode übergangslos in die Wandung des Schlauchstücks einfügt. Die Elektrode wird erzeugt, indem elektrisch leitendes aushärtbares Kunststoffmaterial, vorzugsweise mit Silber oder Kohlenstoff versetzter Silikonkautschuk, in die durch den Blindkörper gegen den Innenraum des Schlauchstückes abgedichtete Öffnung eingegossen oder eingespritzt und mit einem Anschluß an die oben beschriebene Auswerteinheit versehen und anschließend ausgehärtet wird.

Erfindungsgemäß entsteht somit eine Schlauchanordnung, die ein mit Öffnungen in der Schlauchwand versehenes Schlauchstück aufweist, in das vorgefertigte oder in situ hergestellte Elektroden aus leitfähigem Kunststoff in der Weise eingefügt sind, daß das Schlauchstück eine vollkommen einheitliche Innenwandfläche ohne störende Unterbrechungen aufweist.

In einer anderen Ausführungsform der Erfindung wird leitfähiges PVC als Elektrodenelement eingesetzt. Die Leitfähigkeitszelle besteht dann abwechselnd aus Stücken aus isolierendem und elektrisch leitfähigem PVC, die in bekannter Weise miteinander verklebt sind, so daß eine fugenlose Verbindung entsteht.

Diese Vorrichtung ist preiswerter als bekannte Vorrichtungen und kann als Einmal-Artikel in einem Dialysierkreislauf verwendet werden. Da die Vorrichtung vollständig aus biokompatiblem Material gefertigt ist, verläuft der Dialysiervorgang in einer für den Patienten wesentlich schonenderen Weise als bei Verwendung der bisher bekannten Materialien für die Leitfähigkeits-Meßvorrichtung.

Die nachfolgenden Ausführungsbeispiele verdeutlichen die vereinfachte Herstellung einer Leitfähigkeits-Meßvorrichtung mit leitfähigen Polymeren. Dabei wird Bezug genommen auf:
- Figur 1: Ansicht eines erfindungsgemäßen Schlauchstückes mit Öffnungen für die einzusetzenden Elektroden.
- Figur 2: Querschnitt durch ein Schlauchstück mit eingesetzter Elektrode.
- Figur 3: Längsschnitt durch ein Schlauchstück mit eingefügten Elektrodenringen.

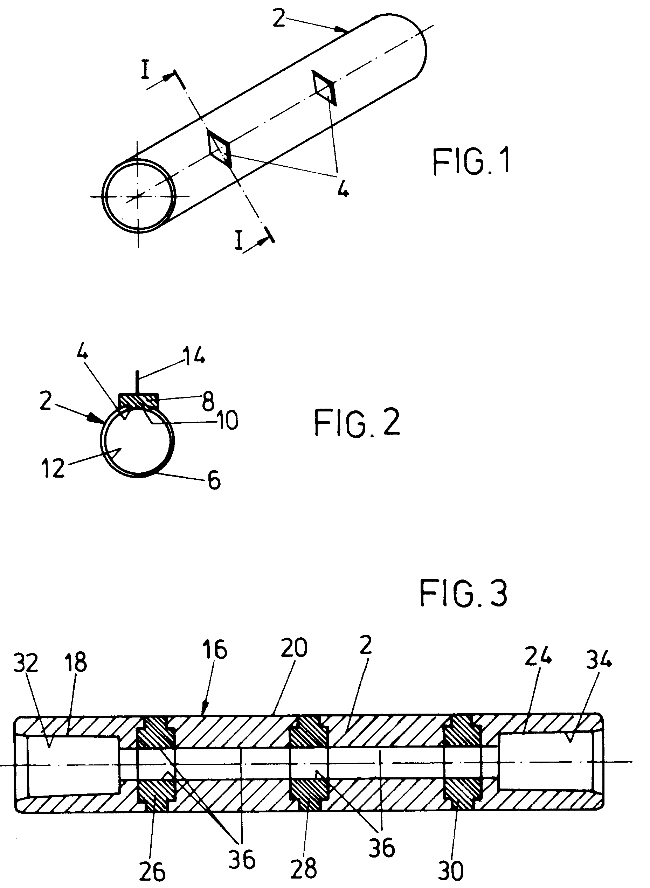

Figur 1 zeigt ein Schlauchstück 2 aus isolierendem Kunststoff, welches Öffnungen 4 für Elektroden aufweist, die die Wandung 6 des Schlauchstücks 2 durchbrechen. Wie die Querschnittsdarstellung des Schlauchstücks 2 (Figur 2) darstellt, ist in die Öffnung 4 ein vorgefertigter Elektrodenformkörper 8, der eine an die Innenwand 12 des Schlauchstückes 2 angepaßte Meßfläche 10 aufweist, mit geeigneten Mitteln (Klebe- bzw. Dichtmittel) flüssigkeitsdicht und paßgenau eingefügt. Das Schlauchstück 2 weist somit eine vollkommen glatte, ununterbrochene Innenwand 12 auf. Der Elektrodenformkörper 8 ist über einen Anschlußdraht 14 mit einer Auswerteinheit zur Erfassung der gemessenen Werte und Umsetzung dieser Werte im Rahmen der Kontrolle des Dialysiervorganges (hier nicht dargestellt) verbunden.

Figur 3 zeigt im Längsschnitt ein rohrförmiges Schlauchstück 16 aus Kunststoff, das abwechselnd aus isolierenden Stücken 18 - 24 und leitfähigen Stücken 26 - 30 besteht, die an ihren Schnitträndern miteinander verklebt sind. Die Endstücke 18 und 24 weisen jeweils ein Lumen 32, 34 auf mit einem Innendurchmesser auf, der vorteilhafterweise größer ist als der Innendurchmesser des Lumens 36 der übrigen Schlauch- oder Elektrodenstücke. Die Endstücke 18 und 24 sind dabei so ausgeführt, daß Schläuche mit einer Innenverklebung eingefügt werden können, wodurch insgesamt eine innen glattes und fugenlose Schlauchanordnung entsteht. Andererseits können die nicht gezeigten Anschlußschlauchstücke mit dem gleichen Innenlumen wie die Stücke 20-22 sowie 26-30 mit Stoß an die Endstücke 18 und 24 angefügt sein, d.h. die Endstücke 18 und 24 haben das gleiche Innenlumen wie die restlichen Stücke.

### Bezugszeichenliste

- 2: Schlauchstück
- 4: Öffnung für Elektrode
- 6: Schlauchwandung
- 8: Elektrodenformkörper
- 10: Meßfläche des Elektrodenformkörpers
- 12: Innenwandfläche des Schlauchstücks
- 14: Anschluß zur Auswerteinheit
- 16: rohrförmiges Schlauchstück
- 18: isolierendes Stück
- 20: isolierendes Stück
- 22: isolierendes Stück
- 24: isolierendes Stück
- 26: leitfähiges Stück
- 28: leitfähiges Stück
- 30: leitfähiges Stück
- 32: Lumen des Abschlußstücks
- 34: Lumen des Abschlußstücks
- 36: Lumen der übrigen Schlauch- oder Elektrodenstücke

## Patentansprüche

1. Schlauchanordnung zum Einsatz für Blutreinigungsverfahren, enthaltend Schlauchstücke (2, 16) mit wenigstens einer Elektrode (8, 26 - 30) zur Bestimmung von Parametern der den Schlauch durchströmenden Flüssigkeiten, dadurch gekennzeichnet, daß die Elektrode (8, 26 - 30) aus einem elektrisch leitenden Kunststoff besteht.

2. Schlauchanordnung nach Anspruch 1, dadurch gekennzeichnet, daß die Elektrode (8) in eine im Schlauchstück (2) vorgesehene Ausnehmung (4) eingesetzt ist.

3. Schlauchanordnung nach Anspruch 2, dadurch gekennzeichnet, daß die Elektrode (8) in der vorgesehenen Ausnehmung im Schlauchstück (2) in situ ausgehärtet ist.

4. Schlauchanordnung nach Anspruch 2, dadurch gekennzeichnet, daß ein ausgeformter und ausgehärteter Elektrodenformkörper (8) formschlüssig in die im Schlauchstück (2) vorgesehene Ausnehmung (4) eingesetzt ist.

5. Schlauchanordnung nach einem der vorangehenden Ansprüche 1 bis 4, dadurch gekennzeichnet, daß das Schlauchstück (2) in Form eines vorgefertigten Rohrstücks mit wenigstens einer Ausnehmung (4) in der Schlauchwand (6) gefertigt ist.

6. Schlauchanordnung nach einem der vorangehenden Ansprüche 1 bis 5, dadurch gekennzeichnet, daß das Schlauchstück (16) mindestens eine ringförmige Elektrode (26 - 30) aus leitfähigem Kunststoff enthält, die fugenlos in das Schlauchstück (16) eingefügt ist.

7. Schlauchanordnung nach Anspruch 6, dadurch gekennzeichnet, daß die Elektrode (26 - 30) aus elektrisch leitfähigem PVC besteht.

8. Schlauchanordnung nach einem der vorangehenden Ansprüche 1 bis 5, dadurch gekennzeichnet, daß der Kunststoff der Elektrode (8, 26 - 30) aus Silikonkautschuk oder PVC ist, und daß das elektrisch leitende Material, in der Elektrode partikelförmiges Silber oder Kohlenstoff ist.
